# EUROPEAN PATENT SPECIFICATION

(11) **EP 0 882 467 B1**
(45) Date of publication and mention of the grant of the patent: **01.10.2003**
(21) Application number: 98116586.3
(22) Date of filing: 03.05.1994
(51) Int. Cl.: A61M 5/315

(54) **Syringe for medicinal purposes**
Medizinische Spritze
Seringue medicale

(30) Priority: 06.05.1993 DE 4314987; 14.09.1993 DE 4331137
(43) Date of publication of application: 09.12.1998
(62) Divisional of application: 94916186.3
(73) Proprietor: Becton Dickinson and Company, Franklin Lakes, NJ 07414-1880 (US)
(72) Inventor: Vetter, Helmut, 88212 Ravensburg (DE); Otto, Thomas, 88267 Vogt (DE); Frasch, Eugen, 88094 Oberteuringen (DE); Bitdinger, Ralf, 38320 Herbeys (FR)
(74) Representative: Selting, Günther, Dipl.-Ing.

(56) References cited:
- EP-A- 0 409 134
- DE-A- 2 945 869
- US-A- 4 267 846
- US-A- 4 711 637
- US-A- 4 883 471
- US-A- 4 946 441

## Description

The invention pertains to a syringe for medicinal purposes, with a syringe barrel, one end of which is designed as an adapter for a cannula or a closure piece, e.g., in the form of a tip cap, and with a plunger arranged in the syringe barrel and movable via a piston rod.

Syringes of this type are increasingly being marketed, especially as ready-to-use syringes in prefilled form, for self-administration by the patient, as has long been the case, especially for regular administration of insulin in diabetics.

In the case of unskilled or infirm people, there is a risk that before application the plunger will accidentally be pulled out of the syringe barrel so that, as a rule, the product is no longer usable.

The features of the preamble of claim 1 are known from US-A-4 883 471.

US-A-4,267,846 discloses a syringe having a piston brake attached to the syringe barrel. The piston brake acts on the piston for controlling the distance in which the piston can move in both inward and outward directions. The syringe barrel has at its proximal end a flange extending perpendicular to the longitudinal axis of the barrel and providing a finger grip for the user of the syringe during its operation. The piston brake has a hook sized and shaped to snap fit over the flange for fastening the piston brake to the barrel. This requires that the barrel has a flange or other protrusion for fastening the piston brake. However, there exist syringes which do not have a flange on the barrel.

It is an object of the invention to provide a syringe having a piston brake, wherein the piston brake is capable of being used in connection with barrels that do not have finger rests.

The present invention is defined by claim 1. Accordingly, the rest piece housing includes a circularly shaped side wall having an inside surface contacting the outer surface of the open proximal end of the barrel and includes two radially outwardly projecting wings, said wings forming a finger rest for applying axial force to the barrel.

In accordance with the invention on the end of the syringe barrel, away from the adapter, a plunger brake is arranged, which is formed by a rest piece surrounding the syringe barrel and attached removably to it, which has a projection extending into the lumen of the barrel, which forms a stop for the side of the plunger away from the adapter. In the plunger rod assembly brake of the present invention the annular piece has two radially extending, diametrically opposite wings that form a finger rest. This is especially advantageous when syringes with very small volumes are used.

The advantage achieved with the invention lies, first of all, in the fact that the projection extending into the lumen of the syringe barrel exerts a braking effect on the plunger, so that even in the case of incorrect manipulation of the syringe by the patient during use, so long as excessive force is not applied, the plunger cannot come out of the syringe barrel.

Additional advantages arise in the course of manufacturing, since during the post-sterilization of solutions prefilled into the syringe barrel, the plunger is frequently forced out of the barrel by the pressure differences that arise. The use of the plunger brake prevents the plunger from being forced out of the barrel, for example, by an air bubble present in the barrel. In this process, the plunger brake can be sterilized along with the unit, if, for example, this is made of polypropylene. As a result, the pharmaceutical safety of the syringe as a whole is increased. Since the plunger brake is placed removably on the syringe barrel, it can be removed from the syringe after use; disposal is simplified by separation of the different materials.

In an advantageous embodiment of the invention, the rest piece is designed as an annular piece, and the projection is designed as fingers extending axially into the barrel, wherein the freely projecting end of the finger forms the stop for the plunger.

In a preferred embodiment of the invention, the annular piece has a separation slit on the side opposite the finger. In this way, the annular piece can especially easily be clipped onto the barrel from the side.

Advantageously, the annular piece is sleeve-shaped, so that it does not interfere with handling of the syringe.

For easier handling, the annular piece can be provided on its outer surface with a riffling travelling in the circumferential direction. This provides additional security when holding or handling the syringe.

In order to also be able to screw the piston rod without difficulty into the plunger, the invention also provides that the piston rod is tapered in the area adjacent to the piston rod thread provided for connection to the plunger.

In the following, the invention will be explained in greater detail, based on exemplified embodiments shown in the drawings, which show:
- Fig. 1: a syringe in accordance with the invention in a preferred embodiment, in side view, with the plunger brake clipped on, and
- Fig. 2: a perspective view of the plunger brake in accordance with Fig. 1.

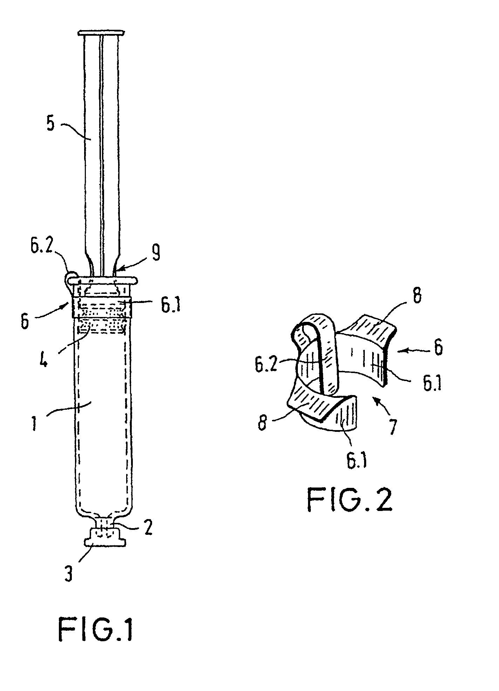

The syringe for medicinal purposes shown in the drawing consists of a syringe barrel 1, one end of which is designed as an adapter 2 for a cannula or - as shown in Fig. 1 - for a closure piece 3 in the form of a tip cap. In the syringe barrel 1, a plunger 4 is arranged, which can be moved with the aid of a piston rod 5.

At the end of the syringe barrel 1 away from the cannula adapter 2, a plunger brake 6 is arranged. The plunger brake 6 consists, in detail, in a first embodiment shown in Figures 1 and 2, of a first annular piece 6.1, surrounding the barrel 1, and a finger 6.2 projecting into the interior of the barrel 1, connected to the annular piece 6.1. Here the free end of the finger 6.2 forms a stop for the plunger 4.

The plunger brake 6 prevents the plunger 4 from being accidentally pulled out of the barrel 1 in the case of incorrect use of the syringe, for example by a unskilled patient.

Also, during the manufacturing process of prefilled, ready-to-use syringes, the plunger brake 6 prevents, for example, the plunger 4 from being forced out of the barrel 1 due to excess pressure occurring in the interior during the post-sterilization of solutions previously filled into the barrel 1.

The annular portion 6.1 of the plunger brake 6, as shown in Fig. 2, consists of a separation slit 7 located on the side opposite the finger 6.2, as a result of which the plunger brake 6 can be clipped especially easily onto the syringe barrel 1 from the side. The annular portion 6.1 itself has an essentially sleeve-like shape, and is thus adapted in shape to the syringe barrel 1.

To improve handling, the annular piece 6.1 can be provided, in a way not shown in detail in the drawing, on its outer surface, with a riffling running in the circumferential direction. In addition, or alternatively. As shown in Fig. 2, the annular piece 6.1 is provided with two radially extending, diametrically opposite wings 8, which form a finger rest. This is especially advantageous in the case of barrels with relatively small volume, since these generally do not have finger rests.

As Fig. 1 shows, the finger 6.2 lies against the inner wall of the syringe barrel 1, so that the piston rod 5 can be moved freely.

The piston rod 5 is tapered in the area 9 adjacent to the piston rod threads, so that the piston rod threads can be screwed into the plunger 4 without further efford, even when the plunger brake 6 is clipped into place.

## Claims

1. A syringe comprising an elongate hollow barrel (1) having a distal end, an open proximal end and a chamber therebetween for retaining fluid, a tip at said distal end of said barrel having a passageway therethrough in fluid communication with said chamber, a plunger rod assembly (4,5) including a piston (4) in slidable fluid tight engagement inside said barrel (1) and an elongate plunger rod (5) connected to said piston (4) and extending proximally through said open end of said barrel (1), a plunger rod assembly brake (6) removably attached to said open proximal end of said barrel (1) including a rest piece housing portion partially surrounding and removably engaging the outer surface of said open proximal end of said barrel (1), a projection on said brake (6) for engaging a complementary protrusion on said plunger rod assembly (4,5) so that proximal movement of said plunger rod assembly (4,5) with respect to said barrel (1) will cause said brake projection and said complementary protrusion to engage and prevent removal of said plunger rod assembly (4,5) from said barrel (1) during normal use of said syringe,
said rest piece housing including a circularly shaped side wall (6.1) having an inside surface contacting the outer surface of the open proximal end of the barrel (1), **characterized in that**
said side wall (6.1) includes two radially outwardly projection wings (8), said wings (8) forming a finger rest for applying axial force to said barrel (1).

2. The syringe of claim 1 wherein said brake projection includes at least one finger (6.2) extending axially into said chamber, said at least one finger (6.2) including a distal end having an end surface for contacting said complementary protrusion of said plunger rod assembly (4,5) to prevent removal of said plunger rod assembly (4,5) from said barrel (1).

3. The syringe of claim 2 wherein said finger (6.2) is in contacting relationship with said barrel (1) in said chamber.

4. The syringe of one of claims 1-3 wherein said circularly shaped side wall (6.1) includes a discontinuity of less than 180°.

5. The syringe of claim 4 wherein said discontinuity is on the opposite side of said brake from said projection.

6. The syringe of one of claims 1-5 wherein said rest piece housing portion is sleeve shaped.

7. The syringe of one of claims 1-6 wherein said complementary protrusion includes a disk-shaped structure on said plunger rod (5) substantially perpendicular to a longitudinal axis of said plunger rod (5).

8. The syringe of claim 7 wherein said disk-shaped structure has a tapered frusto-conically shaped outside diameter.

## Patentansprüche

1. Spritze mit einem länglichen hohlen Zylinder (1), der ein distales Ende, ein offenes proximales Ende und eine dazwischenliegende Kammer zum Halten von Fluid aufweist; einer am distalen Ende des Zylinders angeordnete Spitze mit einem durch diese verlaufenden Durchlass, der in Fluidverbindung mit der Kammer steht, einer Kolbenstangenvorrichtung (4,5), die einen in gleitbarem fluiddichten Eingriff in dem Zylinder (1) angeordneten Kolben (4) und eine längliche Kolbenstange (5) aufweist, welche mit dem Kolben (4) verbunden ist und proximal durch das offene Ende des Zylinders (1) verläuft, einer Kolbenstangenvorrichtungsbremse (6), die abnehmbar an dem offenen proximalen Ende des Zylinders (1) befestigt ist und die einen Raststückhalter aufweist, der die Außenfläche des offenen proximalen Endes des Zylinders (1) teilweise umgibt und abnehmbar an ihr angreift, einem an der Bremse (6) ausgebildeten Vorsprung zum derartigen Angreifen an einer komplementären Vorwölbung der Kolbenstangenvorrichtung (4,5), dass durch eine proximale Bewegung der Kolbenstangenvorrichtung (4,5) relativ zu dem Zylinder (1) der Bremsen-Vorsprung und die komplementäre Vorwölbung zusammengreifen und während der normalen Verwendung der Spritze ein Entfernen der Kolbenstangenvorrichtung (4,5) von dem Zylinder (1) verhindern, wobei der Raststückhalter eine kreisförmige Seitenwand (6.1) mit einer Innenfläche aufweist, welche die Außenfläche des offenen proximalen Endes des Zylinders (1) kontaktiert,
**dadurch gekennzeichnet,**
**dass** die Seitenwand (6.1) zwei radial nach außen verlaufende Vorsprungsflügel (8) aufweist, die eine Fingeranlagefläche zum Ausüben einer Axialkraft auf den Zylinder (1) bilden.

2. Spritze nach Anspruch 1, bei der der Bremsen-Vorsprung mindestens einen Finger (6.2) aufweist, der sich axial in die Kammer erstreckt, wobei der mindestens eine Finger (6.2) ein distales Ende mit einer Endfläche zum Kontaktieren des komplementären Vorsprungs der Kolbenstangenvorrichtung (4,5) aufweist, um ein Entfernen der Kolbenstangenvorrichtung (4,5) von dem Zylinder (1) zu verhindern.

3. Spritze nach Anspruch 2, bei der der Finger (6.2) in der Kammer in Kontaktbeziehung zu dem Zylinder (1) steht.

4. Spritze nach einem der Ansprüche 1-3, bei der die kreisförmige Seitenwand (6.1) eine Diskontinuität von weniger als 180° aufweist.

5. Spritze nach Anspruch 4, bei der die Diskontinuität an der dem Vorsprung gegenüberliegenden Seite der Bremse vorgesehen ist.

6. Spritze nach einem der Ansprüche 1-5, bei der der Raststückhalter hülsenförmig ist.

7. Spritze nach einem der Ansprüche 1-6, bei der der komplementäre Vorsprung eine an der Kolbenstange (5) angeordnete scheibenförmige Struktur aufweist, die im wesentlichen rechtwinklig zu einer Längsachse der Kolbenstange (5) ausgerichtet ist.

8. Spritze nach Anspruch 7, bei der die scheibenförmige Struktur einen sich verjüngenden kegelstumpfförmigen Außendurchmesser hat.

## Revendications

1. Seringue comprenant un corps creux allongé (1) ayant une extrémité distale, une extrémité proximale ouverte et une chambre entre celles-ci pour retenir un fluide, une pointe à ladite extrémité distale dudit corps, traversée par un passage en communication fluide avec ladite chambre, un assemblage de tige de piston (4, 5) comprenant un piston (4) en prise fluide étroite par glissement à l'intérieur dudit corps (1), et une tige de piston allongée (5) connectée audit piston (4), et s'étendant de manière proximale à travers ladite extrémité ouverte dudit corps (1), un frein de l'assemblage de tige de piston (6) fixé de manière amovible à ladite extrémité proximale ouverte dudit corps (1), comprenant une partie de logement de pièce de repos entourant partiellement et en prise de manière amovible avec la surface externe de ladite extrémité proximale ouverte dudit corps (1), une projection dudit frein (6) pour être en prise avec une saillie complémentaire sur ledit assemblage de tige de piston (4, 5), de telle sorte que le mouvement proximal dudit assemblage de tige de piston (4, 5) par rapport audit corps (1) provoquera la prise de ladite projection de frein et de ladite saillie complémentaire, et évitera le retrait dudit assemblage de tige de piston (4, 5) dudit corps (1) au cours d'une utilisation normale de ladite seringue,
ledit logement de pièce de repos comprenant une paroi latérale de forme circulaire (6.1), ayant une surface interne en contact avec la surface externe de l'extrémité proximale ouverte du corps (1),
**caractérisée en ce que**
ladite paroi latérale (6.1) comprend deux ailes à projection externe radiale (8), lesdites ailes (8) formant un repos de doigt pour appliquer une force axiale audit corps (1).

2. Seringue suivant la revendication 1, dans laquelle ladite projection de frein comprend au moins un doigt (6.2) s'étendant axialement dans ladite chambre, ledit au moins un doigt (6.2) comprenant une extrémité distale ayant une surface d'extrémité pour entrer en contact avec ladite saillie complémentaire dudit assemblage de tige de piston (4,5) pour éviter le retrait dudit assemblage de tige de piston (4, 5) dudit corps (1).

3. Seringue suivant la revendication 2, dans laquelle ledit doigt (6.2) est en une relation de contact avec ledit corps (1) dans ladite chambre.

4. Seringue suivant l'une des revendications 1-3, dans laquelle ladite paroi latérale de forme circulaire (6.1) comprend une discontinuité inférieure à 180°.

5. Seringue suivant la revendication 4, dans laquelle ladite discontinuité se trouve sur le côté opposé dudit frein à partir de ladite saillie.

6. Seringue suivant l'une des revendications 1-5, dans laquelle ladite partie de logement de pièce de repos est en forme de manchon.

7. Seringue suivant l'une quelconque des revendications 1-6, dans laquelle ladite saillie complémentaire comprend une structure en forme de disque sur ladite tige de piston (5) essentiellement perpendiculaire à l'axe longitudinal de ladite tige de piston (5).

8. Seringue suivant la revendication 7, dans laquelle ladite structure en forme de disque a un diamètre externe de forme frusto-conique effilée.
